Europäisches Patentamt

European Patent Office

Office européen des brevets

(11) Veröffentlichungsnummer: **0 244 011**
**A2**

(12) # EUROPÄISCHE PATENTANMELDUNG

(21) Anmeldenummer: **87200718.2**

(51) Int. Cl.⁴ **A61B 6/00 , G05D 3/14**

(22) Anmeldetag: **16.04.87**

(30) Priorität: 26.04.86 DE 3614295

(43) Veröffentlichungstag der Anmeldung:
**04.11.87 Patentblatt 87/45**

(84) Benannte Vertragsstaaten:
**DE FR GB IT**

(71) Anmelder: **Philips Patentverwaltung GmbH**
**Wendenstrasse 35 Postfach 10 51 49**
**D-2000 Hamburg 1(DE)**

(84) **DE**

(71) Anmelder: **N.V. Philips' Gloeilampenfabrieken**
**Groenewoudseweg 1**
**NL-5621 BA Eindhoven(NL)**

(84) **FR GB IT**

(72) Erfinder: **Koropp, Norbert**
**Charlottenburger Weg 1**
**D-2057 Reinbek(DE)**

(74) Vertreter: **Hartmann, Heinrich, Dipl.-Ing. et al**
**Philips Patentverwaltung GmbH**
**Wendenstrasse 35 Postfach 10 51 49**
**D-2000 Hamburg 1(DE)**

(54) **Röntgengerät mit Antrieb eines Geräteteils.**

(57) Die Erfindung bezieht sich auf ein Röntgengerät mit einem motorisch angetriebenen Geräteteil. Der Antrieb erfolgt über eine Welle, die in einem Lager gelagert ist, dessen Lagerkräfte durch eine Meßeinrichtung gemessen werden.

Fig.2

EP 0 244 011 A2

## "Röntgengerät mit Antrieb eines Geräteteils"

Die Erfindung betrifft ein Röntgengerät mit Antrieb eines Geräteteils mit einer Meßeinrichtung, die die auf das Geräteteil ausgeübten Kräfte mißt und den Antrieb entsprechend steuert. Solche Röntgengeräte sind bekannt (DE-OS 21 04 509 und DE-OS 23 61 985).

Um dabei zu verhindern, daß der Patient durch das mit der Kraft des Antriebsmotors bewegte Geräteteil eingeklemmt wird oder das Geräteteil gegen den Boden oder die Decke des Untersuchungsraums fährt, muß eine Meßeinrichtung vorgesehen sein, die die auf das Geräteteil ausgeübte Kraft mißt und den Antrieb abschaltet, wenn die auf das Geräteteil ausgeübten Kräfte sich in vorbestimmte Maße ändern. Bei den bekannten Geräten ist der Motorantrieb mit dem Geräteteil über ein Seil verbunden und die Meßeinrichtung erfaßt die auf das Seil wirkenden Kräfte. Dies erfordert, daß bei einem Austausch des Seils auch die Meßeinrichtung - im allgemeinen ein Dehnungsmeßstreifen - ausgetauscht werden muß, da er den Eigenschaften des Seils angepaßt sein muß.

Aufgabe der vorliegenden Erfindung ist es, ein Röntgengerät der eingangs genannten Art so auszugestalten, daß der Antrieb ohne ein Seil erfolgen kan : Diese Aufgabe wird erfindungsgemäß dadurch gelöst, daß der Antrieb über eine Welle erfolgt, die mit wenigstens einem Lager ersehen ist und daß die Meßeinrichtung die auf das Lager in Richtung der Welle ausgeübten Kräfte mißt. Die Antriebswelle überträgt die auf das Geräteteil wirkenden Kräfte auf das Lager, wo sie auf einfache und zuverlässige Weise gemessen werden können.

Eine Ausgestaltung der Erfindung sieht vor, daß das Lager über einen verformbaren Körper mit einem festen Teil des Röntgengerätes verbunden ist und daß der deformierbare Körper mit Sensoren versehen ist, die seine Verformung messen, wobei als Sensor vorzugsweise ein Dehnungsmeßstreifen vorgesehen ist. Der (elastisch) deformierbare vorzugsweise tonnenförmig die Welle umschließende Körper ist einfach herstellbar und bildet zusammen mit einem oder mehreren auf ihn aufgebrachten Dehnungsmeßstreifen ein robustes und zuverlässiges Bauelement.

Eine Weiterbildung der Erfindung sieht vor, daß die Welle mit einem mit dem Antrieb gekoppelten Stirnrad versehen ist, das mit einem mit dem Geräteteil gekoppelten Stirnrad zusammenwirkt. Diese Weiterbildung nutzt die Tatsache aus, daß auf die Schrägzahnstirnräder in Richtung der Welle, auf der sie aufgebracht sind, Kräfte wirken, die der von dem Motorantrieb bzw. dem Geräteteil auf die Welle ausgeübten Kraft proportional sind. Durch Messung dieser Kraft kann das Drehmoment erfaßt und bei Überschreiten eines Grenzwertes der Motorantrieb abgeschaltet werden.

Die Erfindung wird nachstehend anhand der Zeichnung näher erläutert. Es zeigen:

Fig. 1 ein als C-Bogen-Gerät an einem Deckenstativ befestiges Röntgengerät nach der Erfindung,

Fig. 2 einen Teil eines solchen Röntgengerätes,

Fig. 3 ein um eine horizontale Achse umlegbares Röntgengerät, und

Fig. 4 einen Teil eines bei einem Gerät nach Fig. 3 geeigneten Umlegeantriebes.

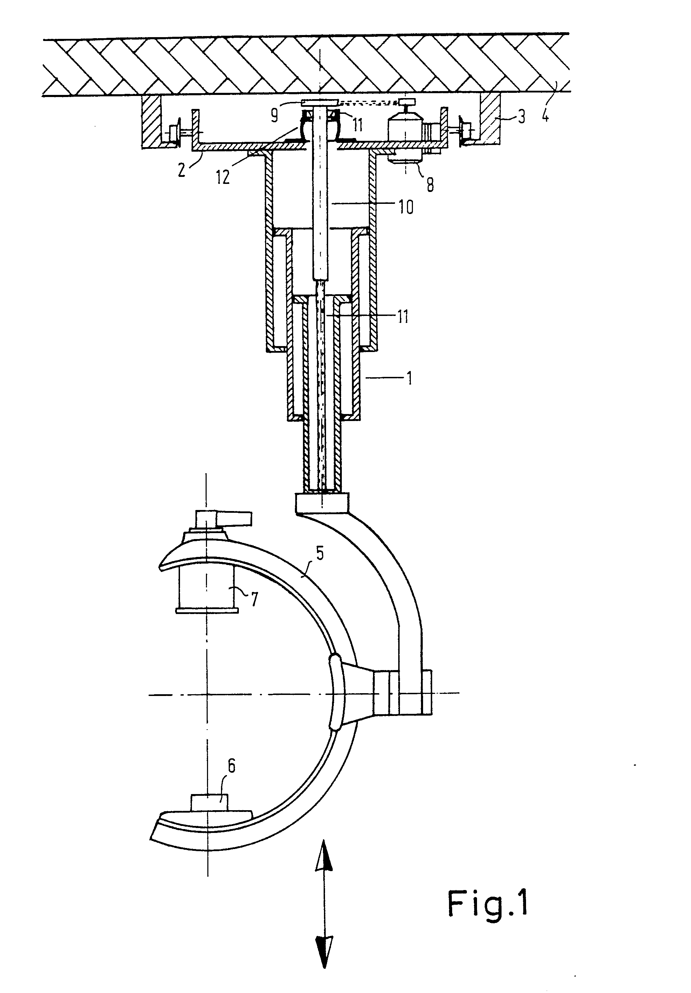

In Fig. 1 ist mit 1 ein Deckenstativ bezeichnet, das mehrere hohlzylindrische und teleskopartig ineinander verschiebbare Teile umfaßt und an einem Stativwagen 2 befestigt ist, der in Schienen 3 an der Decke 4 eines Untersuchungsraumes verfahrbar ist. Das Deckenstativ 1 trägt an einem Rohrbogen 5 einen Röntgenstrahler 6 und einen Bildverstärker 7, die aufeinander ausgerichtet sind. Das Aus-und Einfahren des Deckenstatives erfolgt mit Hilfe eines Motors 8, der auf ein Ritzel 9 einwirkt, das an einer vertikalen Hohlwelle 10 befestigt ist. Die Hohlwelle 10 ist mit einem Innengewinde versehen, in das eine Gewindespindel eingreift, die mit dem untersten Teil des Deckenstatives fest verbunden ist. Je nach Drehrichtung des Motors 8 wird dadurch das Deckenstativ entweder auf-oder abwärts bewegt.

Die Hohlwelle 10 ist in einem Lager 11 gelagert, das über eine tonnenförmige elastische Lagerstütze 12 mit dem Gerätewagen 2 verbunden ist. Die elastische Lagerstütze 12 ist mit einem oder mehreren Dehnungsmeßstreifen versehen, die die Verformung des Lagers messen und den Antriebsmotor 8 still setzen, wenn die Verformung bzw. die von der Last 5...7 auf das Lager ausgeübten Kräfte einen vorgebbaren Wert überschreiten.

Es ist auch möglich, die Gewindespindel 11 und die Hohlwelle 10 miteinander zu vertauschen, so daß die Hohlwelle mit dem unteren Teil des Deckenstatives und die Gewindespindel mit dem Antriebsritzel verbunden ist.

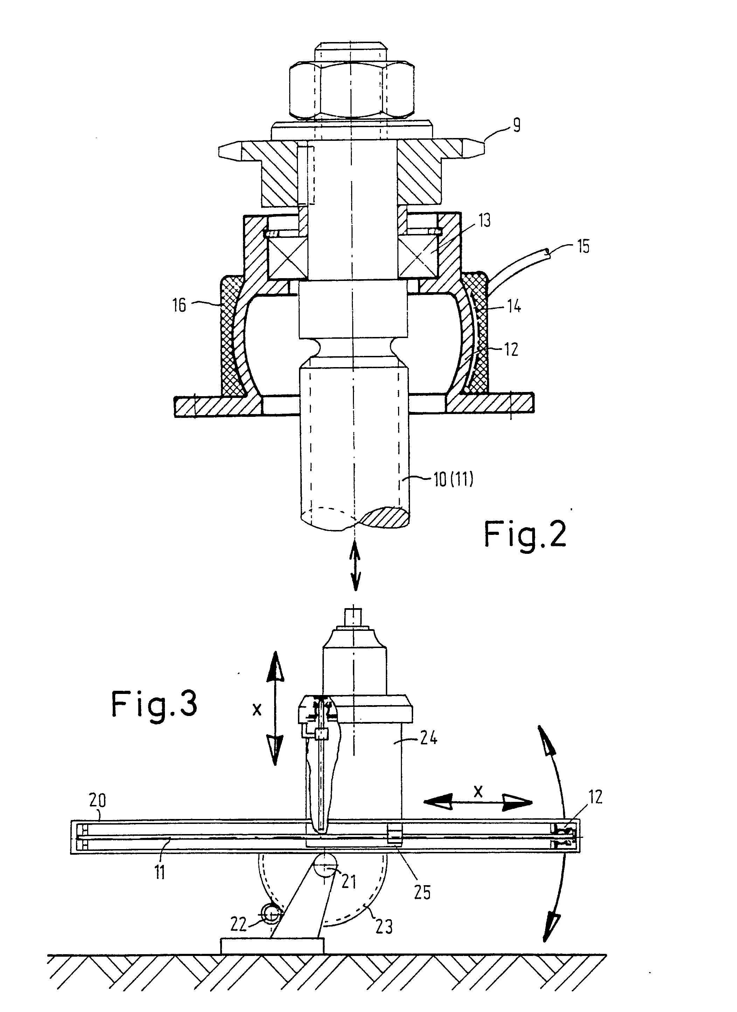

Fig. 2 zeigt die Befestigung der Hohlwelle 10 bzw. der Gewindespindel 11 am Gerätewagen 2. Wie sich daraus ergibt, wird die Welle 10 bzw. 11 von einem Lager 13 getragen, das über die die Welle 10 (11) tonnenförmig umschließende elastische Lagerstütze 12 mit dem in Fig. 2 nicht näher dargestellten Gerätewagen 2 verbunden ist. Auf die elastische Lagerstütze 12 ist wenigstens ein Dehnungsmeßstreifen aufgebracht, mit dessen Hilfe die

Verformung der Lagerstütze I2 in ein elektrisches Signal umgesetzt wird und der über eine Anschlußleitung I5 mit einer Steuerschaltung für den Antriebsmotor versehen ist. Über den bzw. die Meßstreifen ist eine aus einem Elastomer bestehende Schutzhülle I6 gezogen.

Fig. 3 zeigt ein Röntgenuntersuchungsgerät mit einem Patientenlagerungstisch 20, der um eine horizontale Achse 2I mittels eines Antriebsmotors 22 umgelegt werden kann, der auf ein mit der Tischplatte 20 verbundes Zahnsegment 23 einwirkt. Ein Röntgenzielgerät 24 ist mittels der Gewindespindel II, die in einer an dem Zielgerät 24 befestigte Spindelmutter 25 eingreift, in Längsrichtung des Tisches mit Hilfe eines nicht näher dargestellten Elektromotors verfahrbar. Die Gewindespindel ist zumindest an der einen Seite an einem Lager befestigt, das über eine elastische Lagerstütze, wie sie in Fig. 2 dargestellt ist, mit dem Rahmen des Patientenlagerungstisches 20 verbunden ist. Die Verschiebung des Zielgerätes in Kompressionsrichtung, d.h. senkrecht zur Tischplatte, kann - wie aus Fig. 3 ersichtlich - mit Hilfe eines analog gestalteten Antriebs erfolgen.

Die von der Lagerstütze gemessenen Kräfte hängen entscheidend von der Stellung der Tischplatte 20 ab. Sie sind in der in der Zeichnung dargestellten horizontalen Tischplattenstellung minimal und in der vertikalen Stellung der Tischplatte maximal; dazwischen ändern sie sich mit dem Sinus des Schwenkwinkels. Um auch in diesem Fall zusätzlich den Patientenlagerungstisch erfassen zu können, muß das von dem mit der Lagerstütze verbundenen Dehnungsmeßstreifen gelieferte Signal mit einem Signal verglichen werden, das sich sinusförmig mit dem Schwenkwinkel ändert und das beispielsweise einem Sinuspotentiometer entnommen wird, das mit dem Antrieb 22 gekoppelt ist.

Bei Geräten der in Fig. 3 dargestellten Art ist es nicht nur wichtig, die Verschiebebewegungen des Röntgenzielgerätes in Tischlängsrichtung und senkrecht dazu zu überwachen, sondern die Umlegbewegung der Patientenlagerungsplatte, um zu vermeiden, daß die Tischplatte beim Umlegen gegen den Boden bzw. ein darauf befindliches Hindernis stößt.

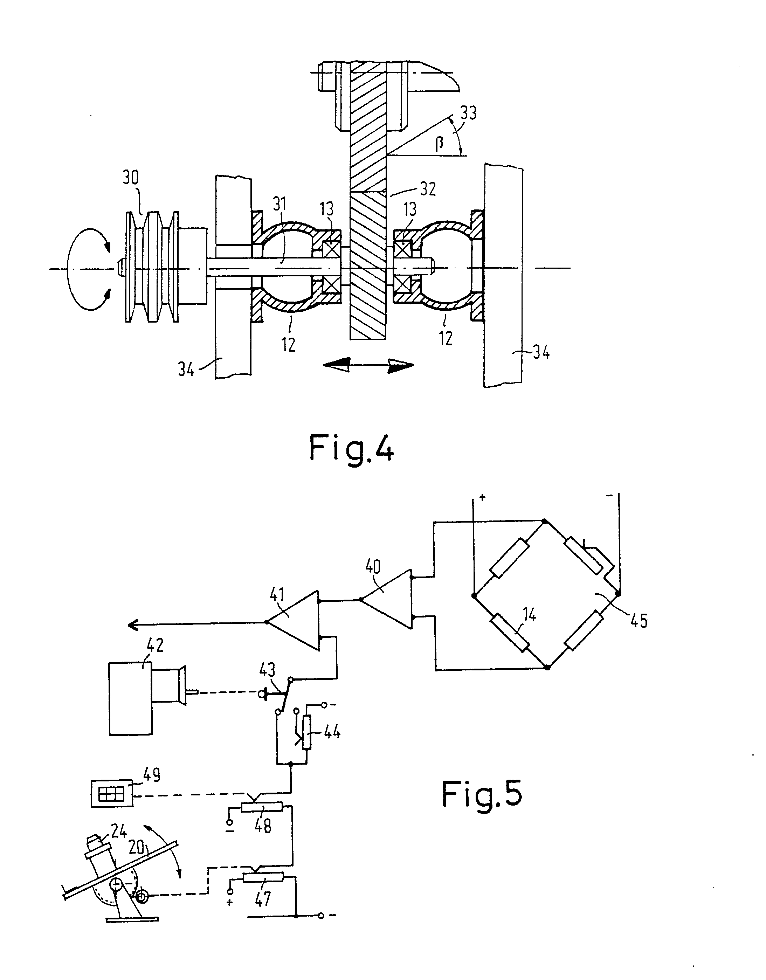

Fig. 4 zeigt einen Teil eines dafür geeigneten Antriebes. Die Motorkraft wird über eine Riemenscheibe 30 auf eine Welle 3I übertragen, auf der sich ein Schrägzahnstirnrad 32 befindet, dessen Flanken unter einem Winkel ß zur Richtung der Welle verlaufen. Das Schrägzahnstirnrad 32 wirkt mit einem weiteren Schrägzahnstirnrad 33 zusammen, das auf nicht näher dargestellte Weise mit dem Zahnsegment 23 (Fig. 3) gekoppelt ist. Das Schrägzahnstirnrad 32 ist auf beiden Seiten in Lagern I3 gelagert, die über die elastischen

Lagerstützen I2 jeweils mit einem in Achsrichtung der Welle 3I festen Teil 34 verbunden sind. Auf die Schrägzahnstirnräder 32 wirkt beim Antrieb eine Schubkraft in Richtung der Welle 3I, deren Größe von dem Drehmoment und deren Richtung von der Drehrichtung abhängt. Sie kann mit den an den Lagerstützen I2 befestigten Sensoren - vorzugsweise Dehnungsmeßstreifen erfaßt werden.

In Fig. 5 ist eine Steuerschaltung zur Beeinflussung des Antriebsmotors dargestellt. Die auf das Geräteteil einwirkenden Kräfte bewirken eine Verformung der elastischen Lagerstütze und dadurch eine Änderung des Widerstandes des Dehnungsmeßstreifens. Diese Widerstandsänderung wird in einer Widerstandsmeßbrücke 45 in eine elektrische Spannung umgesetzt, die von einem Verstärker 40 verstärkt und dem einen Eingang eines Komparators 4I zugeführt wird. Bei einem Gerät, wie dem in Fig. I dargestellten, kann die Spannung am anderen Eingang konstant sein. Soll an dem Gerät 5 bis 7 jedoch ein Zusatzgerät, z.B. eine I00 mm Filmkamera 42, angeschlossen sein, dann muß das dadurch geänderte Gewicht durch eine Änderung der Vergleichsspannung berücksichtigt werden. Dies kann beispielsweise dadurch erfolgen, daß durch einen mit dem Zusatzgerät gekoppelten Schaltkontakt ein Umschalter 43 umgeschaltet wird, der in einer ersten Schaltstellung den Vergleichseingang des Komparators 4I mit der Gleichspannung an einem Potentiometer 44 verbindet und in der anderen Schaltstellung mit der Gleichspannung an einem Abgriff dieses Potentiometers.

Die bei einem Gerät gemäß Fig. 3 in Abhängigkeit von der Schwenkstellung des Gerätes veränderliche Belastung kann mit Hilfe eines weiteren Potentiometers 47 berücksichtigt werden, dessen Abgriff mit dem Umlegeantrieb gekoppelt ist. - Das Patientengewicht, das bei der Umlegung der Patientenlagerungsplatte von Bedeutung ist, kann beispielsweise dadurch berücksichtigt werden, daß die Spannung am Vergleichseingang des Komparators 4I von dem Abgriff eines Potentiometers 48 abgegriffen wird, der entsprechend dem Patientengewicht - z.B. durch eine Tastatur 49 - einstellbar ist.

Die drei Potentiometer 44, 47 und 48 können gegebenenfalls auch in Kaskade geschaltet werden.

## Ansprüche

I. Röntgengerät mit Antrieb eines Geräteteils mit einer Meßeinrichtung, die die auf das Geräteteil ausgeübten Kräfte mißt und den Antrieb entsprechend steuert, dadurch gekennzeichnet, daß der Antrieb über eine Welle erfolgt, die mit wenigstens einem Lager versehen ist und daß die Meßeinrichtung die auf das Lager in Richtung der Welle ausgeübten Kräfte mißt.

2. Röntgengerät nach Anspruch I, dadurch gekennzeichnet, daß das Lager über einen verformbaren Körper mit einem festen Teil des Röntgengerätes verbunden ist und daß der deformierbare Körper mit Sensoren versehen ist, die seine Verformung messen.

3. Röntgengerät nach Anspruch 2, dadurch gekennzeichnet, daß der Körper mit Dehnungsmeßstreifen versehen ist.

4. Röntgengerät nach einem der vorhergehenden Ansprüche, dadurch gekennzeichnet, daß die Welle mit einem mit dem Antrieb gekoppelten Stirnrad versehen ist, das mit einem mit dem Geräteteil gekoppelten Stirnrad zusammenwirkt.

Fig.1

Fig.2

9

13

15

16

14

12

10 (11)

Fig.3

20

11

24

x

x

25

21

22

23

12

Fig.4

Fig.5